# EUROPEAN PATENT APPLICATION

(11) **EP 2 799 104 A1**
(43) Date of publication of application: **05.11.2014**
(21) Application number: 14166563.8
(22) Date of filing: 30.04.2014
(51) Int. Cl.: A61M 21/00

(54) **Apparatus for tactile, visual and aural stimulation of a user**

(30) Priority: 03.05.2013 GB 201308027; 26.11.2013 GB 201320855
(71) Applicant: Hutchings, Nigel Wayne, Haywards Heath, Sussex RH16 2HJ (GB)
(72) Inventor: Hutchings, Nigel Wayne, Haywards Heath, Sussex RH16 2HJ (GB)
(74) Representative: Sweetinburgh, Mark Roger

(57) **Abstract**

Described herein is an apparatus comprising (i) one or more vibration means; (ii) a support for supporting the body of a subject, wherein the one or more vibration means and the support are positioned and configured such that, in use, when a subject is positioned on the support vibrations are transmittable from the vibration means through the support and to the subject; (iii) a light emitting means for providing a visually relaxing experience to a subject positioned on the support; (iv) a sound emitting means for providing an aurally relaxing experience to a subject positioned on the support; (v) a media device; (vi) a control means for coordinating emission of light patterns from the light emitting unit, sound from the sound emitting unit and vibrations from the vibration means with output from the media device; and (vii) an input for a microphone for allowing a user to speak to the subject via the sound emitting means.

## Description

The present invention relates to an apparatus for providing a relaxing experience to a user, in particular to a bed for providing a tactile, visual and aural experience to a user lying on the bed.

Whilst vibroacoustic beds are known, they suffer from a number of drawbacks. For example, they are not generally able to provide an all senses relaxing experience to a user. In addition, it is often difficult for a practitioner to communicate effectively with the user without interrupting their relaxed state.

It is, therefore, an object of the present invention to seek to alleviate the above identified problems.

### SUMMARY OF THE INVENTION

According to one aspect of the present invention, there is provided an apparatus comprising
(i) one or more vibration means;
(ii) a support for supporting the body of a subject, wherein the one or more vibration means and the support are positioned and configured such that, in use, when a subject is positioned on the support vibrations are transmittable from the vibration means through the support and to the subject;
(iii) a light emitting means for providing a visually relaxing experience to a subject positioned on the support;
(iv) a sound emitting means for providing an aurally relaxing experience to a subject positioned on the support;
(v) a media device;
(vi) a control means for coordinating emission of light patterns from the light emitting unit, sound from the sound emitting unit and vibrations from the vibration means with output from the media device;
(vii) an input for a microphone for allowing a user to speak to the subject via the sound emitting means.

Preferably, the one or more vibration means comprise one or more speakers.

Preferably, the support is a mattress or a cushion.

Preferably, the support comprises a bladder filled with water.

Preferably, the bladder filled with water comprises one or more baffles for minimising movement of water within the bladder.

Preferably, the bladder comprises 7 baffles.

Preferably, the support is a water mattress or water cushion.

Preferably, the light emitting means comprises a pair of glasses or goggles.

Preferably, the light emitting means comprises a pair of LED glasses or goggles.

Preferably, the light emitting means comprises a pair of virtual reality glasses.

Preferably, the sound emitting means comprises a pair of headphones.

Preferably, the input for a microphone comprises a socket.

Preferably, the control means comprises a light and sound decoder for converting sound from the media device into relaxing sound emitted from the sound emitting means and relaxing light patterns from the light emitting means.

Preferably, the control means comprises a low frequency filter for transmitting low frequency sound from the media device to the vibration means. Preferably, the low frequency filter means that only low frequency sound is transmitted from the media device to the vibration means.

Preferably, low frequency means below about 200Hz, preferably between about 20Hz and about 200Hz.

Preferably, the control means comprises an amplifier.

Preferably, the control means comprises an audio mixer.

Preferably, the media device comprises an input for receiving data from an external storage device, for example a memory stick.

Preferably, the media device is for playing music.

Preferably, the media device comprises a solid state player.

Preferably, the one or more vibration means are housed in one or more vibration means base units.

Preferably, the one or more vibration means base units are air-tight.

Preferably, the one or more vibration means base units comprise an air-tight box.

Preferably, the control means is housed in a control means base unit.

Preferably, the apparatus comprises a base.

Preferably, the base comprises one or more vibration means base units and a control unit base unit.

Preferably, the base is modular.

Preferably, the apparatus is a bed or a chair.

Preferably, the apparatus comprises a deformable base for the support. Preferably, the apparatus comprises a foam base for the support.

Preferably, the apparatus comprises a waterproof barrier between the support and the one or more vibration means.

Preferably, the apparatus comprises a waterproof barrier between the support and the base.

Preferably, the apparatus comprises a waterproof liner for the support.

Preferably, the apparatus comprises a waterproof liner for the deformable base.

Preferably, the apparatus comprises a frame for accommodating the support.

Preferably, the frame is positioned on top of the base.

Preferably, the frame comprises one or more vibration dampeners for minimising or preventing vibrations being passed from the base to the frame.

Preferably, the base is modular and the frame comprises one or more vibration dampeners for each module of the base.

Preferably, the base comprises one or more vibration means base units and a control unit base unit and the frame comprises one or more vibration dampeners for each of the one or more vibration means base units and control unit base unit.

Preferably, the base comprises one or more recesses for receiving one or more of the one or more vibration dampeners of the frame.

Preferably, the base comprises one or more vibration dampeners for minimising or preventing vibrations being passed from the base to the floor.

Preferably, the frame comprises an overhang, preferably around the base units.

Preferably, the frame comprises a recess for accommodating the outer walls of the base units.

Preferably, the apparatus comprises a heating means for heating the support. Preferably, the heating means is a heat pad.

Preferably, the apparatus comprises a massage means for massaging a subject positioned on the support. Preferably, the massage means is a massage mat.

Preferably, the apparatus comprises one or more lights. Preferably, the lights are provided in the frame, preferably within the overhang of the frame.

Preferably, the apparatus comprises a head rest.

Preferably, the apparatus comprises a face rest.

Preferably, the apparatus comprises a microphone.

Within this specification embodiments have been described in a way which enables a clear and concise specification to be written, but it is intended and will be appreciated that embodiments may be variously combined or separated without parting from the invention.

### DETAILED DESCRIPTION

Example embodiments of the present invention will now be described with reference to the accompanying Figures, in which
Figure 1 shows an exploded perspective view of an apparatus in accordance with the present invention;
Figure 2 shows a schematic wiring diagram for the apparatus shown in Figure 2; and
Figure 3A shows a side view of the base of an apparatus according to the present invention. The two base units and control unit are held together by the mattress frame (not shown) positioned above;
Figure 3B shows a top view of a base unit for use in the present invention;
Figure 3C shows a top view of a frame for use in the present invention positioned over two base units and a control unit;
Figure 3D shows a cross-sectional side view through the perimeter of a frame for use in the present invention;
Figure 3E shows a close up of the view shown in Figure 3D; and
Figure 4 shows a top view of an apparatus of the present invention showing the position of an optional face rest.

The present invention relates to an apparatus, particularly a bed, for providing a relaxing experience to a user.

The principle object of the system is to provide a tactile and sound and light experience for the user to facilitate relaxation and altered states of consciousness. It is also designed to provide hypnotherapists with a unique delivery system to deliver their therapy. Because the client is gently massaged by the sound frequencies as they pass through the water mattress to their body and because the light from the glasses entrains the brain waves to ones of slower and more relaxed state and because the client can hear and feel what is being said through the microphone relaxation is induced that much easier. The aim is to induce a relaxed state of mind and body so hypnotherapy or relaxation can take place.

The base of the present invention preferably comprises three interlocking base units. This makes the apparatus of the present invention much more portable and easier to site than known apparatus.

The base units housing the speakers are preferably sealed. Put another way, it is preferred that they are not vented. This allows much lower frequencies to be emitted from the speakers than would otherwise be the case.

The control unit contains an amplifier, audio unit, audio/visual stimulator and the audio mixer with microphone input.

The apparatus of the present invention enables a user to experience synchronised audio visual stimulation through the audio visual stimulator which is housed in the control unit. The microphone input in the mixer allows the operator/therapist to talk to the user during the user's audio visual stimulation session. This is very useful in hypnotherapy and enables a new therapeutic process to take place called Hypnovibe.

The experience provided by the present invention comprises physical sound wave and physically created vibrations felt through a water mattress via the embedded speakers underneath, music and sounds through the headphone worn by the user and flickering lights seen through the glasses worn by the user. These are all recorded on a memory stick and played using a solid state audio player housed in the control box. When they are played together they create a complete light, sound and tactile sensory immersive experience. The lights and sound and physical vibrations are played at certain frequencies specially chosen and designed to induce a relaxed trance like state. This state can be utilised by a qualified hypnotherapist who may to talk to their client using the microphone. This method has been found to help create the best conditions for therapeutic change work to take place.

As shown in the Figures, a single 220cm x 100cm free flow water mattress 4 is placed on three base units 3a, 3b, 8 (boxes) within a frame 7 positioned over the three base units. Supporting base unit 3a has two sub-woofer speakers 2 housed in an enclosed airtight box. The speakers are facing upwards towards the base of the water mattress 4 so as to send sound through them. Base unit 3b is the same as base unit 3a. These are connected together by a speakon speaker cable (not shown).

Base unit 8 is a control unit and has a built in amplifier 9 and a solid-state player unit 10 within it. It also has a microphone socket 14 within the solid-state player 10. The amplifier 9 has a low frequency bypass option that restricts the sound frequencies to low frequencies. These low frequencies can be transmitted through the speakers 2 and hence through the water mattress 4. A person lays on the water mattress 4 and feels the sound frequencies as they come through the water mattress 4. The user hears a full range of frequencies through headphones 15 that the user wears while lying on the water mattress 4.

Under the water mattress 4 is a range of solenoids that produce physical vibrations through the mattress. This is called a massage matt 18. This massage matt 18 transmits mechanical vibrations through the mattress 4. There is also a heating matt 17 under the mattress 4 to keep the water at a set temperature. The three base units 3a, 3b, 8 support a frame 7 that surrounds the units thus enabling the water mattress 4 to be placed on top of the speaker units 3a, 3b and the control unit 8 and be supported around the edges by the over-hanging frame.

In a drawer 21 in the top of the control unit 8 is a ProNova100 light and sound decoder 12. A pair of light emitting glasses 16 and headphones 15 can be connected to this so the user will see and hear the sounds being sent to it from the solid state player 10.

Music, sound, light and low frequencies are embedded in a sound track that is stored on a USB stick (not shown) that can be played using the solid state player 10 built into the control box.

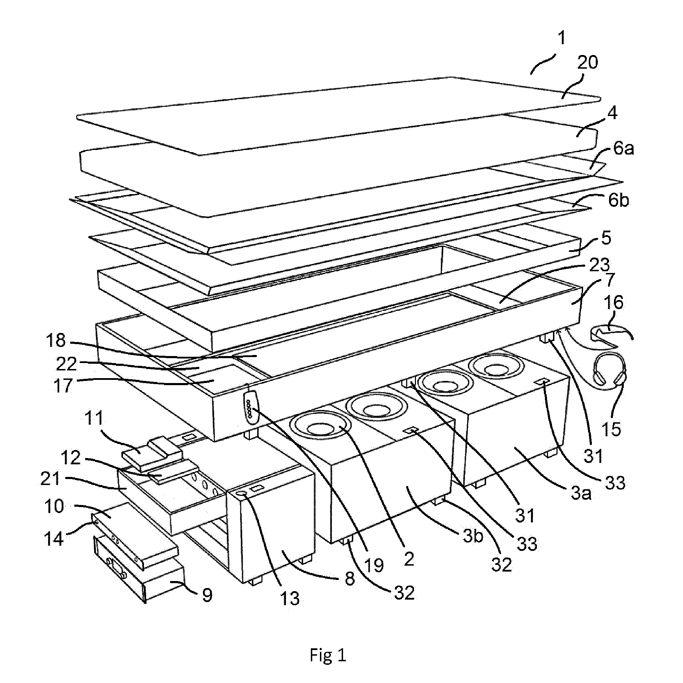

With reference to Figure 1, there is shown an apparatus 1 comprising four speakers 2. Each speaker 2 is a subwoofer for transmitting low frequencies. The speakers 2 are provided in boxes 3a, 3b, with each box containing two speakers. The apparatus includes a water-filled mattress 4 and foam base 5, each provided with a liner 6a, 6b. Liner 6a wraps around the mattress 4. Liner 6b wraps around the foam base 5. The liners 6a, 6b form a double water-proof membrane between the water-filled mattress 4 and the foam base 5.

The mattress 4 is provided with a cushion 20.

The speakers 2 are positioned so that when a person lies on the mattress 4 vibrations are transmittable from the speakers through the foam base 5, through the mattress 4 and the cushion 20 to the person.

The mattress 4, foam base 5 and liners 6a, 6b are held within a frame 7. A plurality of display lights (not shown) are recessed into the underside of the frame 7.

The frame 7 is positioned on top the boxes 3a, 3b and a control unit 8. The control unit 8 includes an amplifier 9, media player 10, audio mixer 11, light decoder 12, and a remote control receiver 13 for the display lights (not shown) in the frame 7. The media player 10 includes a microphone socket 14.

In use, sound from the media player 10 is sent to the audio mixer 11 which is then transmitted to the amplifier 9 which enhances low frequencies within the sound which is then transmitted to the speakers 2. Sound which is sent to the audio mixer is also output therefrom to headphones 15. In order to provide a user with a relaxing visual experience coordinated with vibrations from the speakers 2 and sound from the headphones, sound from the media player 10 is also sent to the light decoder 12 which then transmits relaxing light patterns to a user via glasses 16.

In some embodiments, the glasses 16 are virtual reality glasses. This allows the provision of visual content that can be used for speed learning. For example, it is known that people learn faster when in a relaxed state. If a person is placed in a relaxed state using the apparatus of the present invention and then provided with a range of images and/or words through the glasses 16 and/or headphones 15, this information should be easier to recall at a later date.

The audio mixer 11 and light decoder 12 are housed within a drawer 21.

In order to further enhance the experience provided, a heating pad 17 is provided within the frame 7 underneath the foam base 5 to heat the water in the water-filled mattress 4 to a comfortable and relaxing temperature. In addition, a massage mat 18 is provided under the foam base 5. The massage mat 18 is controlled via a massage mat controller 19.

The frame 7 is provided with a plurality of vibration dampeners 31 for minimising or preventing vibrations being passed from the boxes and control unit to the frame. The frame 7 includes a pair of vibration dampeners for each box 3a, 3b and the control unit 8 of the base. In this respect, it will be appreciated that there are also vibration dampeners projecting from the base of the frame adjacent the far side of the frame shown in Figure 1 forming pairs with the vibration dampeners projecting from the base of the frame adjacent the near side of frame shown in Figure 1.

The boxes 3a, 3b and control unit 8 comprises recesses 33 for receiving the vibration dampeners 31 of the frame.

The boxes 3a, 3b and control unit also comprise their own vibration dampeners 32 for minimising or preventing vibrations being passed from the boxes and control unit to the floor. In the embodiment shown, a vibration dampener 32 is provided at each corner of the base of each of the boxes 3a, 3b and control unit 8.

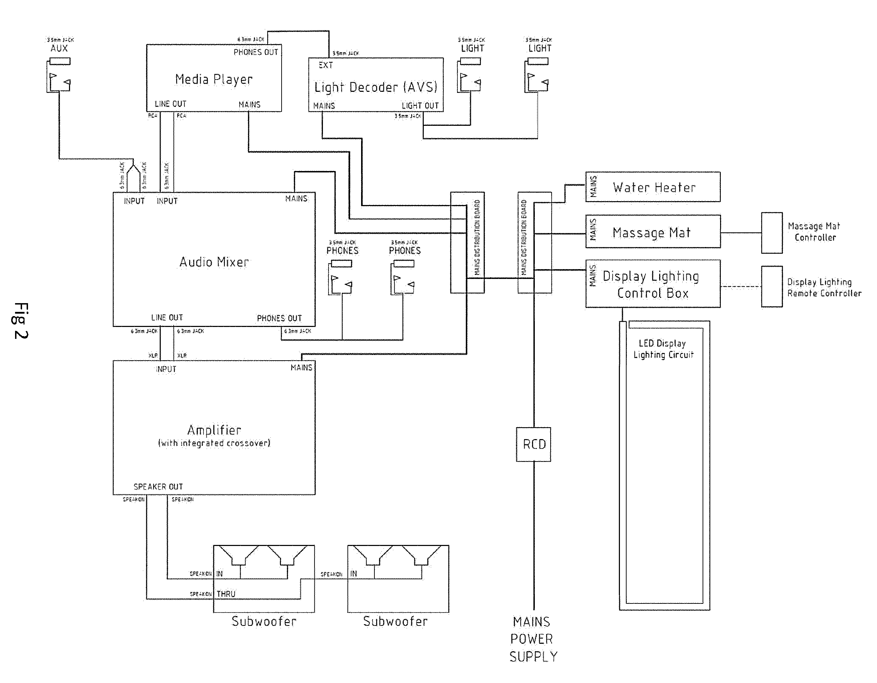

With reference to Figure 2, a wiring/connection diagram is shown for the apparatus shown in Figure 1.

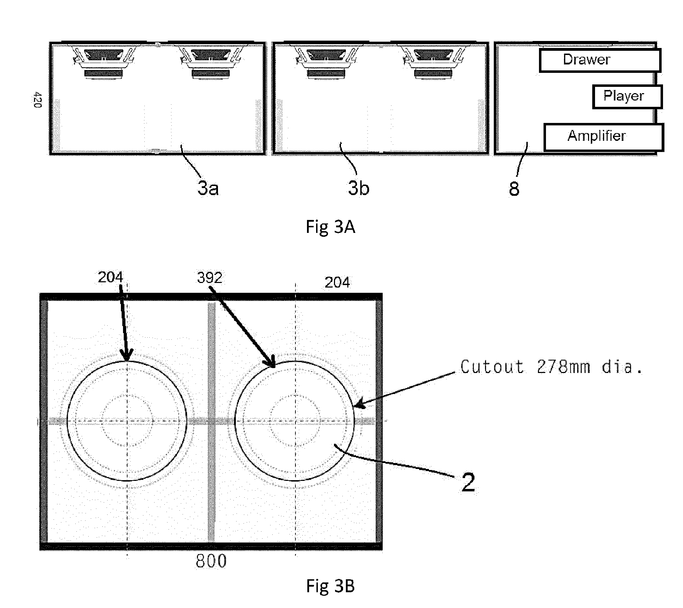

With reference to Figure 3A and 3B, an arrangement of base units and control unit is shown.

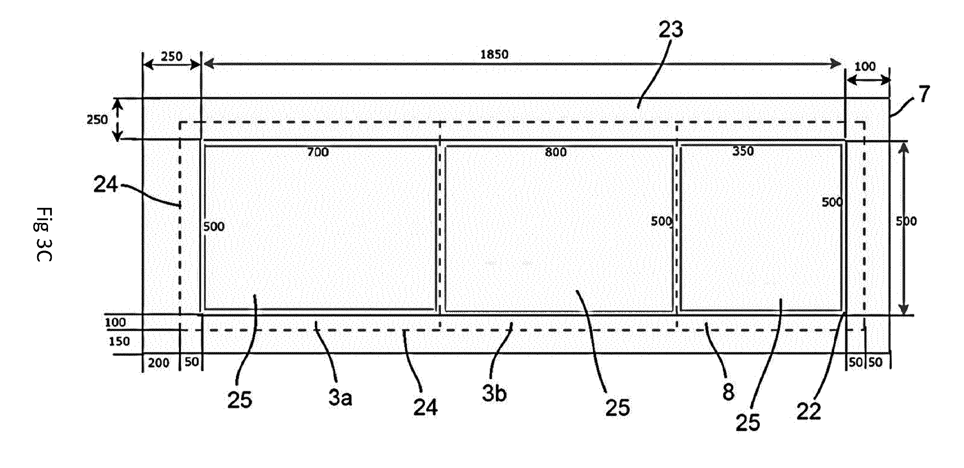

Figure 3C shows a top view of a frame 7 positioned over the two base units 3a, 3b and control unit 8 of Figure 3A. The base units 3a, 3b and control unit 8 are shown with dashed lines. The frame 7 includes an aperture 22 surrounded by a perimeter 23. The perimeter 23 sits on top of the external walls 24 of the base units 3a, 3b and control unit 8. Within the aperture 22, each of the base units 3a, 3b and control unit 8 are provided with an overlay panel 25 for protecting the speakers 2

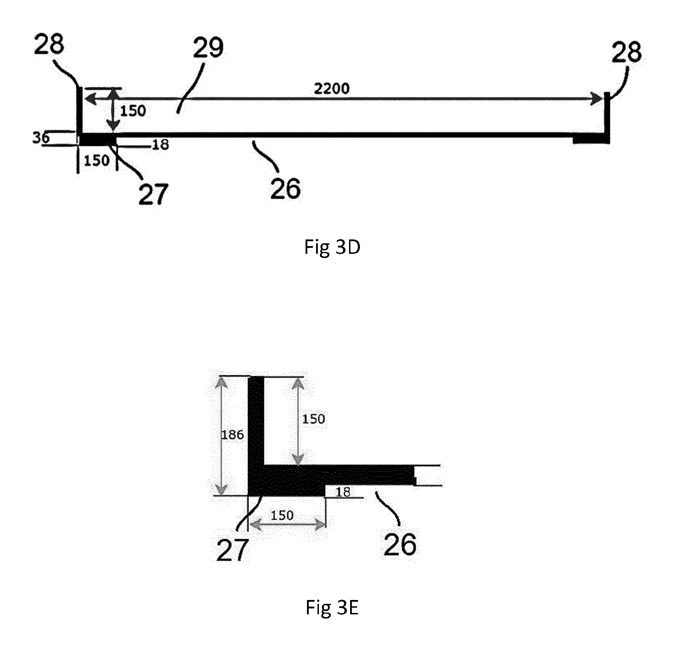

As shown in Figures 3D and 3E, the frame includes a recess 26 for accommodating the base units 3a, 3b and control unit 8 with an overhang 27 which is positioned around the base units 3a, 3b and control unit 8.

The frame 7 includes walls 28 forming a recess 29 for accommodating the foam base, liners 6a, 6b, water mattress 4 and cushion 20.

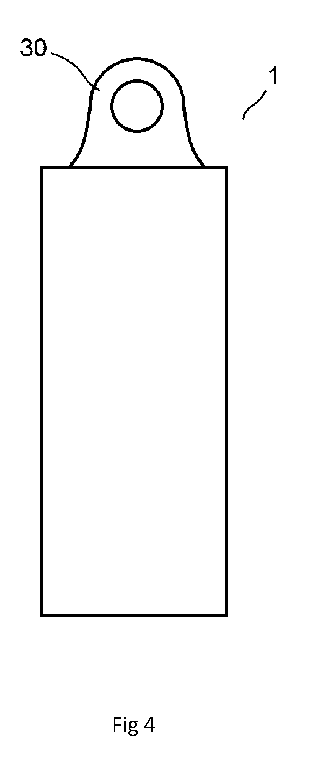

As shown in Figure 4, a portable face rest 30 can be provided, for example attached to the end of the end box 3a to support the head of a client who is having massage. The face rest may comprise face holes in much the same way as those provided on a massage couch.

The present invention provides a sound, tactile and light delivery system with a built in control unit for mixing the sounds and light. This is seen via LED light emitting glasses, heard via headphone and felt via four sub-woofers housed in two separate air-tight cabinets. The loudspeaker enclosures have the speakers facing upwards underneath the water mattress so the sound vibrations go through the water to the client lying on top of it.

While there may be other units that use vibroacoustic technology none have incorporated all the elements mentioned in one structure. None have a three-box unit that fits together. None have two separate units as enclosed and airtight boxes housing two subwoofer speakers in each box. None have a separately constructed platform to house the water mattress, which fits over the three boxes holding the whole structure together. None have a control unit as one of the three boxes that houses the amplifiers, solid-state player and light and sound decoder and mixer.

The invention includes the following embodiments.
1. An apparatus comprising
   (i) one or more vibration means;
   (ii) a support for supporting the body of a subject, wherein the one or more vibration means and the support are positioned and configured such that, in use, when a subject is positioned on the support vibrations are transmittable from the vibration means through the support and to the subject;
   (iii) a light emitting means for providing a visually relaxing experience to a subject positioned on the support;
   (iv) a sound emitting means for providing an aurally relaxing experience to a subject positioned on the support;
   (v) a media device;
   (vi) a control means for coordinating emission of light patterns from the light emitting unit, sound from the sound emitting unit and vibrations from the vibration means with output from the media device;
   (vii) an input for a microphone for allowing a user to speak to the subject via the sound emitting means.
2. An apparatus according to embodiment 1, wherein the one or more vibration means comprise one or more speakers.
3. An apparatus according to embodiment 1 or 2, wherein the support is a mattress or a cushion.
4. An apparatus according to any preceding embodiment, wherein the support comprises a bladder filled with water.
5. An apparatus according to any preceding embodiment, wherein the light emitting means comprises a pair of glasses or goggles.
6. An apparatus according to any preceding embodiment, wherein the sound emitting means comprises a pair of headphones.
7. An apparatus according to any preceding embodiment, wherein the control means comprises a light and sound decoder for converting sound from the media device into relaxing sound emitted from the sound emitting means and relaxing light patterns from the light emitting means.
8. An apparatus according to any preceding embodiment, wherein the control means comprises a low frequency filter for transmitting low frequency sound from the media device to the vibration means.
9. An apparatus according to any preceding embodiment, wherein the media device comprises an input for receiving data from an external storage device, for example a memory stick.
10. An apparatus according to any preceding embodiment, wherein the one or more vibration means are housed in one or more vibration means base units.
11. An apparatus according to embodiment 10, wherein the one or more vibration means base units are air-tight.
12. An apparatus according to any preceding embodiment, wherein the control means is housed in a control means base unit.
13. An apparatus according to any preceding embodiment, comprising a base.
14. An apparatus according to embodiment 13, wherein the base comprises one or more vibration means base units and a control unit base unit.
15. An apparatus according to any preceding embodiment, comprising a deformable base for the support.
16. An apparatus according to any preceding embodiment, comprising a waterproof barrier between the support and the one or more vibration means.
17. An apparatus according to any preceding embodiment, comprising a frame for accommodating the support.
18. An apparatus according to embodiment 17, wherein the frame is positioned on top of the base.
19. An apparatus according to embodiment 17 or 18, wherein the frame comprises an overhang.
20. An apparatus according to any of embodiments 17 to 19, wherein the frame comprises a recess for accommodating a base of the apparatus.
21. An apparatus according to any preceding embodiment, comprising a heating means for heating the support.
22. An apparatus according to any preceding embodiment, comprising a massage means for massaging a subject positioned on the support.
23. An apparatus according to any preceding embodiment, comprising one or more lights.
24. An apparatus according to any preceding embodiment, comprising a headrest.
25. An apparatus substantially as hereinbefore described with reference to the accompanying Figures.

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications are covered by the appended claims.

## Claims

1. An apparatus comprising
(i) one or more vibration means;
(ii) a support for supporting the body of a subject, wherein the one or more vibration means and the support are positioned and configured such that, in use, when a subject is positioned on the support vibrations are transmittable from the vibration means through the support and to the subject;
(iii) a light emitting means for providing a visually relaxing experience to a subject positioned on the support;
(iv) a sound emitting means for providing an aurally relaxing experience to a subject positioned on the support;
(v) a media device;
(vi) a control means for coordinating emission of light patterns from the light emitting unit, sound from the sound emitting unit and vibrations from the vibration means with output from the media device; and
(vii) an input for a microphone for allowing a user to speak to the subject via the sound emitting means.

2. An apparatus according to claim 1, wherein the one or more vibration means comprise one or more speakers.

3. An apparatus according to claim 1 or 2, wherein (i) the support is a mattress or a cushion, and/or (ii) wherein the support comprises a bladder filled with water.

4. An apparatus according to any preceding claim, wherein the light emitting means comprises a pair of glasses or goggles, and/or wherein the sound emitting means comprises a pair of headphones.

5. An apparatus according to any preceding claim, wherein the control means comprises a light and sound decoder for converting sound from the media device into relaxing sound emitted from the sound emitting means and relaxing light patterns from the light emitting means, and/or wherein the control means comprises a low frequency filter for transmitting low frequency sound from the media device to the vibration means.

6. An apparatus according to any preceding claim, wherein the media device comprises an input for receiving data from an external storage device, for example a memory stick.

7. An apparatus according to any preceding claim, wherein (i) the one or more vibration means are housed in one or more vibration means base units, optionally wherein the one or more vibration means base units are air-tight, and/or (ii) wherein the control means is housed in a control means base unit.

8. An apparatus according to any preceding claim, comprising a base, optionally, wherein the base comprises one or more vibration means base units and a control unit base unit.

9. An apparatus according to any preceding claim, comprising a deformable base for the support, and/or a waterproof barrier between the support and the one or more vibration means.

10. An apparatus according to any preceding claim, comprising a frame for accommodating the support, optionally (i) wherein the frame is positioned on top of the base, and/or (ii) wherein the frame comprises an overhang, and/or (iii) wherein the frame comprises a recess for accommodating a base of the apparatus.

11. An apparatus according to any preceding claim comprising a frame and a base, wherein the frame comprises one or more vibration dampeners for minimising or preventing vibrations being passed from the base to the frame.

12. An apparatus according to claim 11, wherein the base comprises one or more recesses for receiving one or more of the one or more vibration dampeners of the frame.

13. An apparatus according to any preceding claim, comprising a heating means for heating the support.

14. An apparatus according to any preceding claim, comprising a massage means for massaging a subject positioned on the support.

15. An apparatus according to any preceding claim, comprising one or more lights, and/or a headrest.
